(19) Europäisches Patentamt
European Patent Office
Office européen des brevets

(11) Numéro de publication: **0 072 299**
**B1**

(12) # FASCICULE DE BREVET EUROPÉEN

(45) Date de publication du fascicule du brevet: **17.12.86**

(51) Int. Cl.⁴: **C 07 D 237/20**, C 07 D 413/12, A 61 K 31/50

(21) Numéro de dépôt: **82401423.7**

(22) Date de dépôt: **30.07.82**

(54) **Nouveaux dérivés de la méthyl-4 phényl-6 pyridazines actifs sur le système nerveux central.**

(30) Priorité: **10.08.81 FR 8115435**

(43) Date de publication de la demande: **16.02.83 Bulletin 83/7**

(45) Mention de la délivrance du brevet: **17.12.86 Bulletin 86/51**

(84) Etats contractants désignés: **AT BE CH DE FR GB IT LI LU NL SE**

(56) Documents cité:
**FR-A-2 141 697**

**CHEMICAL ABSTRACTS, vol. 76, 1972, page 22, no. 41974h, Columbus, Ohio, USA, M.R. Ornellas: "Biochemical studies of cerebral subfractions after chronic administration of 4-methyl-3- 2-(morpholino)ethylamino -6-phenylpyridazine hydrochloride, AG 620"**

(73) Titulaire: **SANOFI, société anonyme, 40, Avenue George V, F-75008 Paris (FR)**

(72) Inventeur: **Wermuth, Camille, 3 rue de la Côte d'Azur, F-67100 Strasbourg (FR)**
Inventeur: **Davi, Horace, Le Saut des Champs Murles, F-34980 St. Gely du Fesc (FR)**
Inventeur: **Bizière, Katleen, 6 Lotissement Rayons d'Oc, F-34170 Clapiers (FR)**

(74) Mandataire: **Gillard, Marie- Louise, Cabinet Beau de Loménie 55, Rue d'Amsterdam, F-75008 Paris (FR)**

**EP 0 072 299 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

Depuis de nombreuses années, des dérivés de la pyridazine ont été proposés en tant que médicaments. Dans un grand nombre de cas, il s'agit de substances actives sur le système cardiovasculaire et présentant en particulier un effec hypotenseur ou vasodilatateur. Plus rarement on a mentionné parmi les dérivés de la pyridazine une action antiinflammatoire et analgésique. Enfin, FR-A-2 141 697 décrit une famille de produits répondant à la formule:

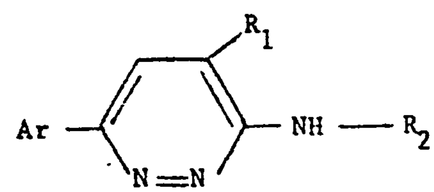

où
$R_1$ représente l'hydrogène ou un groupe alkyle inférieur Ar représente un reste aromatique
$R_2$ désigne un groupe

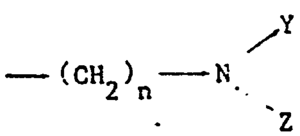

dans lequel n = 2 ou 3
et Y et Z représentent un groupe alkyle inférieur ou bien 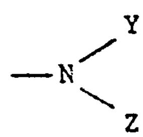

conatitue un radical hétérocyclique.
Ces composés sont caractérisés par une action psychotrope de type psychostimulante.
Une étude ultérieure du composé où $R_1 = CH_3$,
Ar = phényle et $R_2$ =

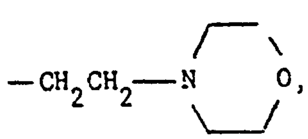

qui a reçu la Dénomination Commune Internationale "Minaprine", a permis de montrer qu'il s'agit d'une action psychotrope d'un type nouveau que l'on a désignée par activité "désinhibitrice". Par ailleurs à doses supérieures à 100 mg/kg per os, ce produit se montre convulsivant.

On a maintenant trouvé que certaines phényl-6 méthyl-4 amino-3 pyridazines possèdent les mêdmes propriétés pharmacologiques et biochimiques que la minaprine tout en étant moins toxiques et pratiquement dépourvues d'action convulsivante.

La présente invention a donc pour objet une famille de dérivés de la pyridazine répondant à la formule générale (I):

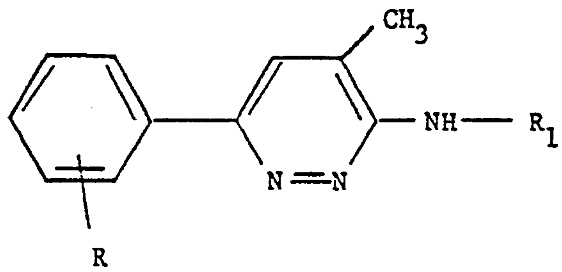 (I)

dans laquelle
R est H ou OH
$R_1$ représente H ou le groupe

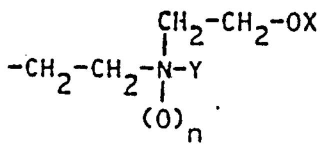 .

où
n = 0 ou 1
X = H
Y = H ou alkyle inférieur (1 à 4 atomes de carbone) ou bien X et Y pris ensemble forment un groupe éthylène ou oxoéthylène, avec les conditions suivantes:
1) lorsque n = 0 et X + Y est un groupe ethylène R est un groupe hydroxy;
2) lorsque n = 1 X + Y est un groups éthylène

La présente invention concerne également les sels d'addition d'acides des composés de formule (I).

Elle comprend également un procédé de préparation des divers composés de formule (I) ainsi que leur application en thérapeutique.

Les composés (I) sont obtenus de façon générale à partir d'une chloro-3 methyl-4 pyridazine substituée en 6 par un groupe phényle éventuellement subsitué

Quand $R_1$ est

Quand $R_1$ est 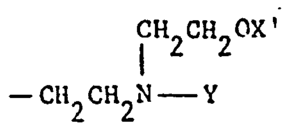 , on fait agir sur la chloropyridazine un composé 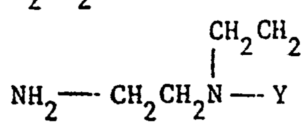 , dans lequel Y

dans lequel Y a la signification indiquée précédemment et X' reptésente l'hydrogéne ou bien X' et Y représentent ensemble un groupe éthylène.

La réaction entre le dérivé chloré et l'amine s'effectue en général par chauffage au sein d'un solvant convenable tel qu'un alcool, le plus souvent à température d'ébullition du solvant. La durée de la réaction varie de quelques heures à plusieurs jours suivant la nature des réactifs mis en jeu. Lorsque la réaction s'avère trop lente elle peut être catalysée par addition d'une petite quantité de poudre de cuivre.

On effectue la réaction en présence d'un accepteur d'hydracide destiné à fixer l'acide chlorhydrique formé dans la réaction. Le plus souvent on utilise comme tel un excès de l'amine.

L'isolement du composé (I) s'effectue par reprise dans l'eau et extraction par un solvant convenable tel que l'acétate d'éthyle.

Quand X et Y pris ensemble représentent un groupe oxoéthylène, on a accès aux composés (I) correspondants à partir du composé (I) dans lequel X et Y sont égal à X.

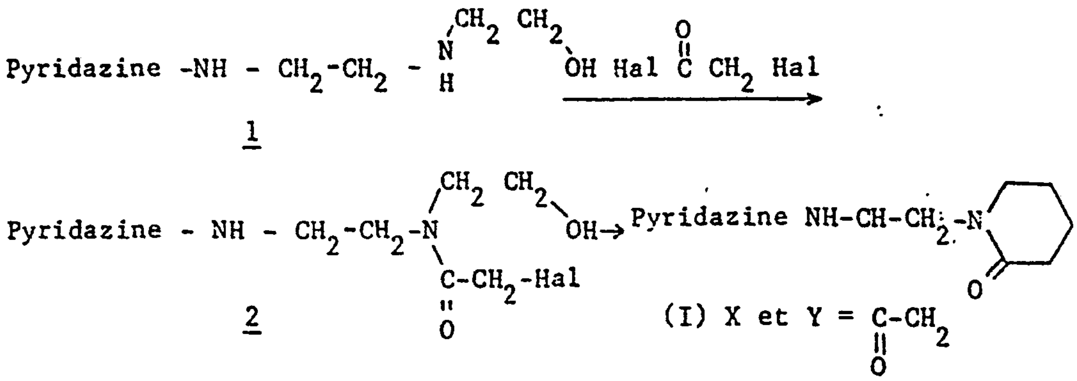

1

2

(I) X et Y =

Par action d'un halogénure d'haloacétyle au sein d'un solvant inerte tel que le dichlorométhane et en présence d'un agent alcalin tel que la soude aqueuse à une température comprise entre -10 et 0°C on obtient l'amine tertiaire 2. Celle-ci, par action d'un alcoolate alcalin tel que le méthylate de solium au sein du méthanol à la température d'ébullition du solvant conduit au composé (I) dans lequel X et Y représentent un groupe oxoéthylène. Celui-ci est isolé sous forme d'un sel tel que le chlorhydrate.

De même en remplaçant l'halogénure d'haloacétyle par le bromacétate d'éthyle et en opérant au reflux d'un solvant tel que le diméthylformamide en présence d'une base organique telle que le triéthylamine, on obtient l'amine tertiaire 3.

Après saponification de la fonction ester en milieu acide, par exemple par l'acide chlorhydrique un obtient directement le composé attendu (I) [X et Y représentant

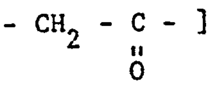

isolé sous forme de sel de l'acide utilisé.

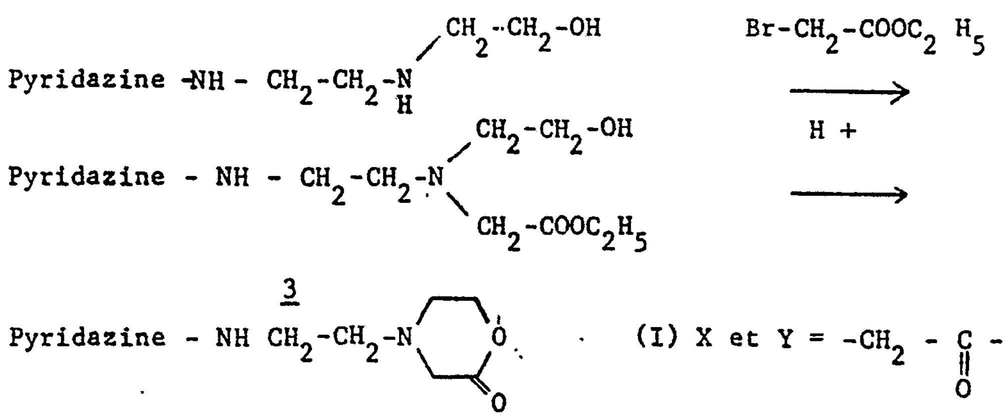


3

(I) X et Y =

Dans le cas où n = 1, on obtient les composés (I) à partir du composé correspondant où n = 0 par oxydation notamment par action d'un peracide tel que l'acide para-chloro-perbenzoïque au sein d'un solvant inerte tel que le chloroforme et à une température ne dépassant pas 20°C.

Lorsque $R_1$ = H, il n'est pas possible d'obtenir directement l'amino-3 pyridazine à partir du dérivé chloré correspondant.

Dans ce cas on passe par l'intermédiaire du dérivé hydrazino-3 obtenu par action au reflux d'un large excès d'hydrazine sur le dérivé chloré correspondant. Celui-ci, hydrogéné en présence de nickel de Raney au sein

d'un solvant convenable conduit au dérivé correspondant (I) $R_1$ = H.

Enfin, dans tous les cas, lorsque le phényleen 6 du cycle de la pyridazine est substitué par un groupe OH (R = OH), il est préférable de préparer selon les méthodes indiquées précédemment le composé correspondant (I) où le groupe phényle porte un substituent alcoxy en même position puis à désalkyler ce composé par une méthode connue par exemple par action de l'acide bromhydrique au sein de l'acide acétique au reflux.

Les composés (I) ainsi obtenus peuvent être salifiés de façon classique par action de l'acide sur une solution chaude de la base le solvant étant choisi de façon que le sel cristallise par refroidissement.

Les chloro-3 pyridazines utilisées comme produit de départ sont obtenues à partir des 2H pyridazones-3 correspondantes per action d'un excès d'oxychlorure de phosphore.

Les 2H pyridazones 3 sont connues ou peuvent être obtenues par des procédés connus tels que l'action de l'hydrazine sur des acides γ-cétoniques ou des dérivés de ceux-ci.

Les exemples suivants sont donnés à titre d'illustration de la présente invention.

**Exemple 1**

[(hydroxy-2 éthylamino)-2 éthylamino)-3 méthyl-4 phényl-6 pyridazine, dichlorhydrate (CM 30311)

R = H; $R_1$ = $CH_2CH_2NH$--$CH_2CH_2OH$

On porte à reflux pendant 48 h un mélange de 7,7g de chloro-3 méthyl-4 phényl-6 pyridazine et 15g de N-(hydroxy-2 éthyl) éthylène-diamine dans 50 ml de n-butanol en présence de 2g de poudre de cuivre. On verse le mélange réactionnel dans 100 ml d'eau et extrait avec de l'éther.

On extrait la phase éthérée avec une solution d'acide sulfurique 5N la phase aqueuse est alcalinisée par addition de soude en pastilles.

On essore les cristaux et recristallise dans le mélange éther isopropylique-isopropanol. F: 91°C.

Dichlorhydrate: On dissout 3,9g de la base dans l'isopropanol chaud puis on ajoute 2,44 ml de solution concentrée d'acide chlorhydrique; par refroidissement le chlorhydrate cristallise. On essore et recristallise dans l'isopropanol. F: 144°C.

En opérant comme dans l'exemple 1, mais en remplaçant la N-(hydroxy-2 éthyl)-éthylène-diamine par une quantité équivalente de $N_1$-éthyl $N_1$ (hydroxy-2 éthyl)-éthylènediamine, on obtient de la même façon la {[N-éthyl (hydroxy-2 éthylamino)]-2 éthylamino}-3 méthyl-4 phényl-6 pyridazine (SR 95084) isolée sous forme de dichlorhydrate; produit très hygroscopique se décomposant à partir de 140°C.

**Exemple 2**

[(oxo-3 morpholinyl-4)-2 éthylamino]-3 méthyl-4 phényl-6 pyridazine, chlorhydrate (CM 30488)

R = H ; $R_1$ = $-CH_2CH_2-$ 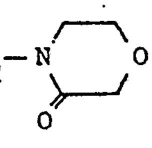

On dissout 5,4 g de CM 30311 (exemple 1) dans 100 ml de dichlorométhane puis on ajoute une solution de 8g de soude dans 100 ml d'eau. On refroidit le mélange à -10, -5°C et ajoute goutte à goutte sous bonne agitation 2,2 g de chlorure de chloracétyle.

On poursuit l'agitation pendant 12 h puis on évapore à siccité. On dissout le résidu dans 50 ml de méthanol anhydre et ajoute une solution de méthylate de sodium obtenue par action de 0,46 g de sodium sur 50 ml de méthanol. On chauffe au reflux durant 6 h puis on évapore à siccité. On reprend le résidu dans l'eau et extrait avec de l'acétate d'éthyle.

On sèche la solution et évapore à siccité, on obtient une huile.

Chlorhydrate: on dissout la base dans le minimum d'isopropanol chaud et ajoute un équivalent de solution aqueuse concentrée d'acide chlorhydrique. On ajoute de l'éther et laisse cristalliser. F: 190-191°C.

**Exemple 3**

[(oxo-2 morpholinyl-4)-2 éthylamino]-3 méthyl-4 phényl-6 pyridazine, dichlorhydrate (CM 30489)

R = H ; $R_1$ = 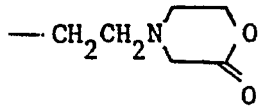

On dissout 2,7 g de CM 30311 (exemple 1) dans 50 ml de diméthylformamide et on ajoute 1 g de triéthylamine et 1,7 g de bromacétate d'éthyle. On porte au reflux pendant 1 h. On ajoute de l'eau alcalinise et extrait avec de l'acétate d'éthyle. On évapore le solvant à siccité et chromatographie sur colonne de silice. En éluant avec de l'acétate d'éthyle, on obtient une huile jaunâtre.

On dissout 1 g de cette huile dans 20 ml de solution aqueuse d'acide chlorhydrique 3 N et porte à reflux pendant 14 h. On lave la solution avec de l'éther, puis on évapore à siccité la phase aqueuse. Le résidu cristallise dans l'éther F: 170-172° C.

**Exemple 4**

(morpholino-2 éthylamino)-3 méthyl-4 (hydroxy-4 phényl)-6 pyridazine, dibromhydrate (CM 30366)

R = 4-OH ; $R_1$ = 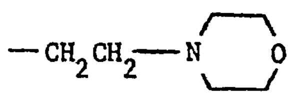

a) (morpholino-2 éthylamino)-3 méthyl-4 (méthoxy-4 phényl) 6 pyridazine.

On opère comme dans l'exemple 1 en remplaçant la N-(hydroxy-2 éthyl)-éthylènediamine par une quantité équivalente de morpholino-2 éthylamine et la chloro-3 méthyl-4 phényl-6 pyridazine par une quantité correspondante de chloro-3 méthyl-4 (méthoxy-4 phényl)-6 pyridazine.

De la même façon on isole le produit sous forme de dichlorhydrate. F: 225° C.

b) CM 30366

On porte au reflux pendant 6 h, 19 g de la base libérée du chlorhydrate obtenu ci-dessus dans 150 ml d'un mélange 2-1 (vol/vol) d'acide bromhydrique 48 % et d'acide acétique.

On évapore à siccité. Il reste une huile brune qui cristallise dans un mélange éthanol-éther. On essore les cristaux et recristallise dans l'éthanol à 95; F: 162° C.

En opérant comme dans l'exemple 4 a) à partir des chloro-3 méthyl-4 (méthoxy-2 ou -3 phényl)-6 pyridazines puis en déméthylant les produits ainsi obtenus selon la méthode de l'exemple 4 b), on obtient respectivement:

- (le morpholino-2 éthylamino)-3 méthyl-4 (hydroxy-2 phényl)-6 pyradazine (SR 95070) isolé sous forme de dibromhydrate;

F: 170° C (décomposition);

- (le morpholino-2 éthylamino)-3 méthyl-4 (hydroxy-3 phényl)-6 pyridazine (SR 95082) isolé sous forme de dibromhydrate;

F: 180° C avec décomposition.

**Exemple 5**

N-oxyde de (morpholino-2 éthylamino)-3 méthyl-4 phényl-6 pyridazine (CM 30490)

(I) R = H ; $R_1$ = 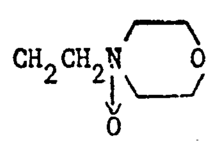

On dissout 1,5 g de morpholino-2 éthylamino-3 méthyl-4 phényl-6 pyridazine dans 100 ml de chloroforme anhydre. On ajoute 0,90 g d'acide para-chloro-perbenzoïque et laisse pendant 48 h à température ambiante. On

lave la solution organique avec une solution aqueuse de bicarbonate de sodium à 10 % On sèche la solution et évapore à siccité; le résidu cristallise par addition d'éther. On recristallise dans le mélange acétate d'éthyle-hexane.

On obtient 1 g de cristaux. F. avec décomposition à partir de 150° C.

**Exemple 6**

Amino-3 méthyl-4 phényl-6 pyridazine

(chlorhydrate) (CM 30465)

(I) $R = R_1 = H$

a) Hydrazino-3 méthyl-4 phényl-6 pyridazine

On porte au reflux le mélange de 5 g de chloro-3 méthyl-4 phényl-6 pyridazine et 12 ml d'hydrate d'hydrazine. Après 1 h 30 min on laisse refroidir le milieu réactionnel. Il se sépare un solide qu'on essore et lave avec un peu d'eau. On recristallise dans le mélange isopropanol-éther isopropylique,poids 4,5 g F.: 162° C.

b) CM 30465

On dissout 6,5 g du dérivé précédent dans le minimum de méthanol et ajoute 2,5 g de nickel de Raney. On hydrogène sous une pression de 5 atmosphères pendant 48 h. On filtre le catalyseur et évapore à siccité. Le résidu est recristallisé dans un mélange isopropanol-éther isopropylique. Poids 5,25 g F.: 130-2° C.

Chlohydrate: A 2 g de base dissoute dans le minimun d'isopropanol on ajoute 1,2 équivalent d'acide chlorhydrique gazeux puis on précipite par addition d'éther. On obtient une poudre blanche (1,7 g), F: 172-4° C.

En opérant comme dans l'exemple 6 a) à partir de chloro-3 méthyl-4 (métioxy-4 phényl)-6 pyridazine, on obtient le dérivé hydrazino-3 correspondant. Celui-ci traité comme dans l'exemple 6 b) fournit le dérivé amino-3 correspondent.

Enfin par déméthylation comme dans l'exemple 4 b) on obtient l'amino-3 méthyl-4 (hydroxy-4 phényl)-6 pyridazine (SR 95087) isolé sous forme de bromhydrate; F.: 260° C (décomposition).

Les produits selon l'invention ont été soumis à des essais pharmacologiques en vue de déterminer leur activité sur le système nerveux central et leur toxicité.

Toxicité aigue

Les produits à étudier ont été administrés par voie intrapéritonéale à doses croissantes à des lots de 10 souris. La mortalité provoquée par les produits étudiés a été notée pendant les 24 h ayant suivi l'adminsitration du produit.

A partir des résultats obtenus on détermine, pour chacun des produits étudiés la dose létale 50 c'est-à-dire la dose provoquant la mort de 50 % des animaux étudiés.

Pendant les mêmes expériences on note également la dose seuil convulsivante du produit c'est-à-dire la dose minimale pour laquelle commence à se manifester une activité convulsivante.

Les résultats obtenus sont réunis dans le tableau I. Dans ce tableau on a fait figurer à titre de comparaison 2 produits décrits dans FR-A-2 141 697 cité ci-dessus.

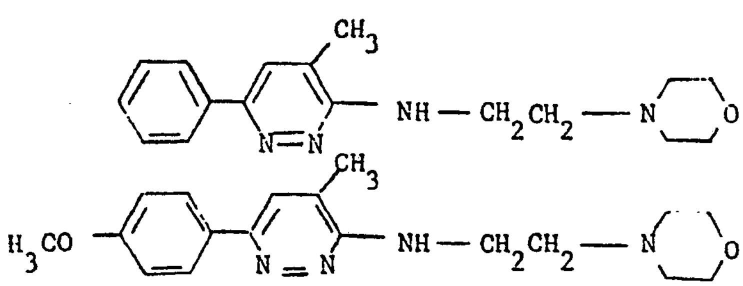

Minaprine (DCI)

CM 30073

Tableau I

| Composé | $DL_{50}$ (mg/kg ; i.p.) | Dose seuil convulsivante (mg/kg ; i.p.) |
|---|---|---|
| Minaprine | 63 (52-77) | 35 |
| CM 30073 | 19 (11-35) | 5 |
| CM 30311 | > 200 | 200 |
| CM 30366 | > 200 | 200 |
| SR 30488 | > 300 | 300 |
| SR 30465 | 226 | 200 |
| SR 30490 | > 200 | 300 |
| SR 95070 | > 200 | 200 |
| SR 95082 | > 200 | > 200 |
| SR 95084 | $100 < DL_{50} < 200$ | 200 |
| SR 95087 | > 200 | > 200 |

Les chiffres figurant dans le tableau I montrent que les produits selon l'invention présentent une toxicité et une action convulsivante très inférieures à celles des produits de référence.

Activité antidépressive

- Reaction de désespoir

Ce test a été réalisé chez la souris femelle CDI (Charles River) pesant 18 à 23 g selon la méthode décrite par Porsolt (Archives Internationales de Pharmacodynamie, 1977 229, 327-336).

Le principe de ce test est le suivant: lorsqu'une souris est placée dans un récipient étroit rempli d'eau, elle se débat puis au bout de 2 à 4 min elle s'immobilise et flotte sur le ventre le dos arrondi, les pattes postérieures ramenées sous le corps et elle ne fait que quelques mouvements nécessaires pour se maintenir la tête hors de l'eau. C'est la réaction dite de désespoir (despair réaction).

Certains psychotropes notamment les antidépresseurs allongent le temps pendant lequel la souris se débat.

Le protocole suivant a été choisi:

Les produits à étudier ont été administrés i.p. 1 h avant le test. Pour le test les animaux sont placés dans un récipient étroit (10 x 10 x 10 cm) rempli d'eau sur une hauteur de 6 cm, la température de l'eau étant de 24° C ± 2° C. Les animaux sont laissés 6 min dans l'eau et on mesure le temps où l'animal reste immobile entre la 2e et

la 6e min. Plus ce temps est court, plus la substance est active.

Chaque substance a été étudiée sur un lot de 10 souris. Les résultats sont la moyenne d'au moins deux expériences.

- Antagonisme de la ptôse induite par la réserpine

Ce test décrit par GOURET (Journal de Pharmacologie (Paris) 1973 4 (1), 105-128) a été réalisé chez la souris femelle CDI (Charles River) pesant 20 ± 1 g. La réserpine entraîne un ptôsis 1 heure après son administration intraveineuse; certains antidépresseurs s'opposent à ce ptôsis.

Le protocole suivant a été choisi: les substances à étudier ont été administrées i.p. La réserpine est administrée simultanément par voie intraveineuse à la dose de 2 mg/kg. 1 heure après l'administration de réserpine on note le nombre d'animaux ne présentant pas de ptôsis.

Ce test a été réalisé sur des lots de 10 souris les résultats sont exprimés en pourcentage d'animaux ne présentant pas de ptôsis et sont la moyenne d'au moins deux expériences.

Les résultats obtenus avec les produits de l'invention figurent dans le tableau II. A titre de comparaison on a également indiqué les résultats obtenus avec les deux produits de l'art antérieur minaprine et CM 30073.

**Tableau II**

**Activite antidepressive**

| Composés | Antagonisme de la ptôse induite par la réserpine ($DE_{50}$, mg/kg; i.p.) | "Behavioral Despair" % de réduction de la durée d'immobilisation |
|---|---|---|
| Minaprine | 5 (4-7) | 10 mg/kg : - 35% ** |
| CM 30073 | 1 mg/kg : 30%<br>5 mg/kg : 60%<br>produit trop toxique aux doses supérieures | —— |
| CM 30366 | 12 (11-15) | 10 mg/kg : - 31% ** |
| CM 30465 | 8,6 (8,3-9) | 10 mg/kg : - 24% ** |
| SR 30488 | 20 (13-31) | —— |
| SR 30490 | ≃ 50 | —— |

Activité dopaminomimétique

L'activité dopaminomimétique des produits de l'invention a été étudiée sur les récepteurs dopaminergiques striataux de la souris selon la technique décrite par P. PROTAIS et J. COSTENTIN Journal de Pharmacologie (Paris) 7, 251-255 (1976).

La lésion unilatérale des neurones dopaminergiques nigrostriataux induit une hypersensibilité des récepteurs de la dopamine au niveau du striatum. L'asymétrie qui en résulte est révélée par des rotations de l'animal dans le sens contralatéral aux récepteurs les plus intensément stimulés.

Après administration des produits à étudier par voie intrapéritonéale on compte pendant une période de 2 min le nombre de tours effectués par l'animal.

On exprime les résultats sous forme de pourcentage des variations par rapport aux témoins n'ayant pas reçu de produit à étudier.

Les résultats obtenus avec divers produits de l'invention sont réunis dans le tableau III où figurent également les résultats des deux produits de comparaison: minaprine et CM 30073.

**Tableau III**

| Composés | Doses µmoles/kg; i.p. | Nombre moyen de rotations ipsilatérales en 2 min % par rapport aux témoins |
|---|---|---|
| Minaprine | 5,3 | - 91% |
| CM 30073 | 5,3 | 0% |
| CM 30311 | 5,3 | - 146% |
| CM 30366 | 5,3 | - 111% |
| CM 30465 | 5,3 | - 89% |
| CM 30488 | 5,3 | - 90% |
| CM 30489 | 5,3 | - 97% |
| SR 95070 | 5,3 | - 89% |

Des tableaux I, II et III, il ressort que les composés représentatifs de la présente invention ont sur le plan global une activité antidépressive et dopaminomimétique du même ordre de grandeur que celle de la minaprine.

Par rapport à la minaprine et surtout au CM 30073 les produits représentatifs de la présente invention sont très peu toxiques et pratiquement dépourvus d'activité convulsivante.

Ainsi les nouveaux composés de la présente invention peuvent être utilisés en thérapeutique dans tous les troubles du comportement psychomoteur.

Ils peuvent être prescrits entre autres dans l'hyperkinésie chez l'enfant dans la dépression masquée de l'adulte dans les états dépressifs sérieux dans la dépression du vieillard ainsi que dans les troubles de la mémoire et de la sénescence.

Ces produits peuvent être administrés par voie orale ou par voie injectable. Les compositions thérapeutiques peuvent être solides ou liquides et se présenter par exemple sous forme de comprimés, gélules, granulés suppositoires ou préparations injectables.

La posologie peut varier dans de larges proportions, en particulier suivant le type et la gravité de l'affection à traiter et suivant le mode d'administration. Chez l'adulte par voie orale elle est le plus souvent comprise entre 0,010 g et 0,500 g éventuellament répartie en plusieurs prises.

A titre d'exemple on peut indiquer la préparation galénique suivante:

COMPRIMES

CM 30465 200 mg
Cellulose microcristalline 100 mg
Lactose 197 mg
Stéarate de magnésium 3 mg
500 mg

**Revendications**

**pour les Estats contractants (BE, CH, DE, FR, GB, IT, LI, LU, NL, SE)**

1. Dérivés de la méthyl-4 phényl-6 pyridazine de formule:

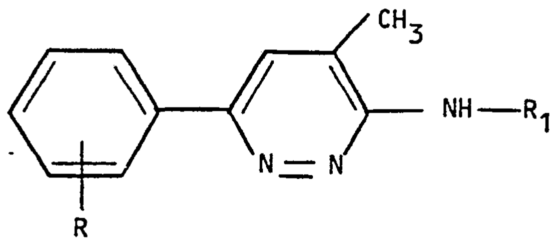

(I)

dans laquelle
R est H ou OH
$R_1$ représente H ou le groupe

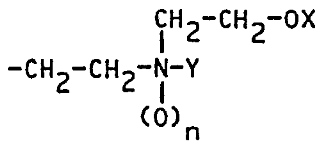

où
n = 0 ou 1
X = H
Y = H ou alkyle inférieur (1 à 4 atomes de carbone)
ou bien X et Y pris ensemble forment un groupe éthylène ou oxoéthylène, avec les conditions suivantes:
1) lorsque n = 0 et X + Y est un groupe éthylène R est un groupe hydroxy
2) lorsque n = 1 X + Y est un groupe éthylène.

2. Dérivés selon la revendication 1, caractérisés en ce qu'ils sont les sels d'addition des composés de formule avec un acide pharmaceutiquement acceptable.

3. Procédé de préparation de dérivés de la méthyl-4 phényl-6 pyridazine de formule:

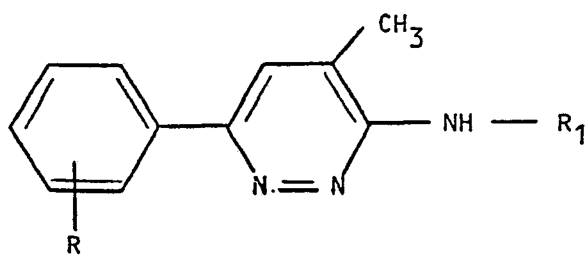

(I)

dans laquelle
R est H ou OH
$R_7$ représente le groupe

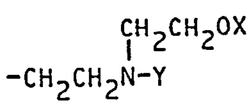

dans lequel: X est H, Y est H ou alkyle inférieur ou bien X et Y représentent ensemble un groupe éthylène lorsque R est OH, caractérisé en ce que l'on fait réagir la chloro-3 méthyl-4 pyridazine substituée en 6 par un groupe phényle éventuellement substitue avec un composé de formule ,

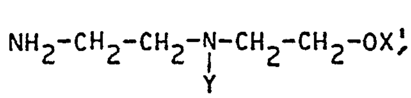

dans laquelle Y est tel que défini ci-dessus, et X'représente l'hydrogéne ou bien X' et Y représentent ensemble un groupe éthylène, la réaction étant effectuée par chauffage des réactifs dans un solvant

convenable tel qu'un alcool, à une température voisine de la température d'ébullition dudit solvant et en présence d'un accepteur de l'acide chlorhydrique formé.

4. Procédé selon la revendication 3, caractérisé en ce que la réaction est effectuée en présence de cuivre utilis comme catalyseur.

5. Procédé de préparation de dérivés de la méthyl-4 phenyl-6 pyridazine de formule:

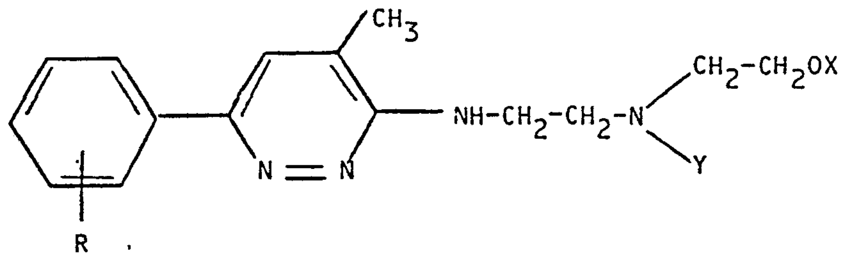

dans laquelle

R est H ou OH

X et Y représentent ensemble un groupe oxoéthylène, caractérisé en ce que l'on prépare un produit de formule (I), dans laquelle X et Y sont égaux à H en utilisant le procédé selon l'une des revendications 3 et 4 et que l'on fait réagir ledit produit sur un halogénure d'haloacétyle de formule

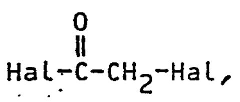

dans laquelle Hal est un halogène, ladite réaction étant effectuée dans un solvant inerte en présence d'un agent alcalin et à une température comprise entre -10 et 0°C et que l'on traite l'amine tertiaire obtenue en milieu méthanol avec un alcoolate alcalin.

6. Procédé de préparation de dérivés de la méthyl-4 phényl-6 pyridazine de formule:

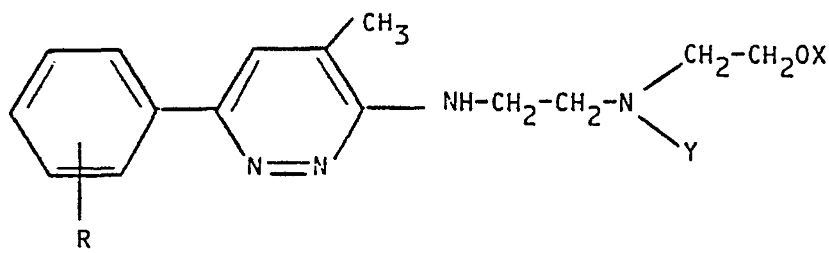

dans laquelle

R est H ou OH

X et Y représentent ensemble un groupe oxoéthylène, caractérisé en ce que l'on prépare un produit de formule (I), dans laquelle X et Y sont H selon l'une des revendications 3 et 4 et que l'on fait réagir ce produit avec le bromacétate d'éthyle, la réaction étant effectuée à reflux dans un solvant tel que le dimethylformamide et en présence d'une base organique telle que la triéthylamine et que l'on saponifie en milieu acide l'amine tertiaire obtenue.

7. Procédé de préparation de dérivés de la méthyl-4 phényl-6 pyridazine de formule:

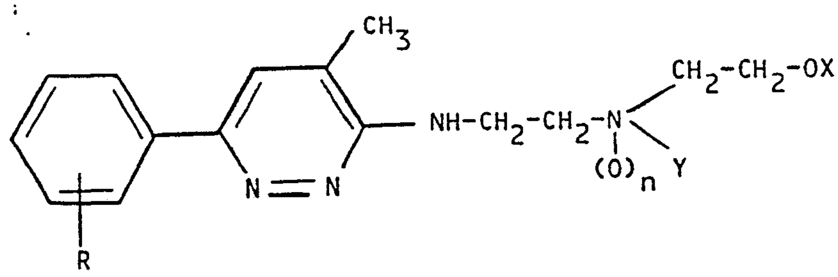

dans laquelle
R est H ou OH
n est 1
X et Y representent ensemble un groupe éthylène, caractérisé en ce que l'on utilise comme produit de depart un produit de formule (I) préparé selon l'une des revendications 3 et 4 et que l'on traite ledit produit par un peracide tel que l'acide parachloroperbenzoïque, cette réaction étant effectuée dans un solvant inerte à une température inférieure à environ 20°C.

8. Procédé pour la préparation d'un dérivé de la méthyl-4 phényl-6 pyridazine de formule:

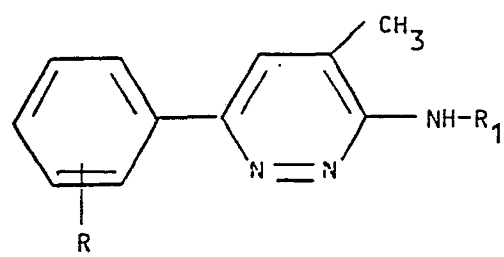

dans laquelle
R est H ou OH, et $R_1$ est H
caractérisé en ce que l'on fait réagir le produit de formule:

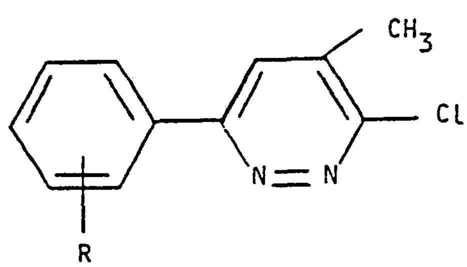

avec un large excès d'hydrazine de façon à obtenir le dérivé hydrazino-3 de la pyridazine et on hydrogène avec du nickel de Raney dans un solvant convenable le produit obtenu,

9. Médicaments utilisables notamment pour le traitement des états dépressifs, caractérisés en ce qu'ils contiennent au moins un produit selon l'une des revendications 1 et 2.

10. Médicaments selon la revendication 9, caractérisés en ce qu'ils contiennent de 0,01 g à 0,500 g d'au moins un produit selon les revendications 1 et 2.

**Revendications**

**pour l'Etat contractant AT**

1. Procédé de préparation de dérivés de la méthyl-4 phényl-6 pyridazine de formule:

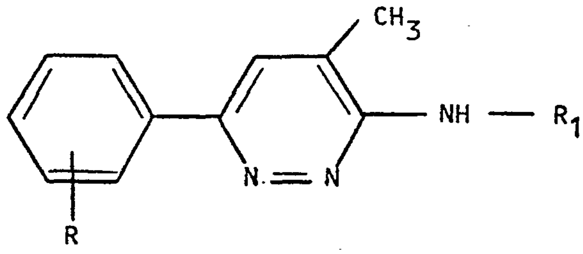

(I)

dans laquelle
R est H ou OH
$R_1$ représente le groupe

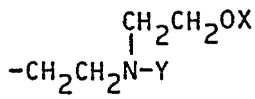

dans lequel: X est H, Y est H ou alkyle inférieur ou bien X et Y représentent ensemble un groupe éthylène lorsque R est OH, caractérisé en ce que l'on fait réagir la chloro-3 méthyl-4 pyridazine substituée en 6 par un groupe phényle éventuellement substitué avec un composé de formule

FIG35/20

dans laquelle Y est tel que défini ci-dessus, et X'represente l'hydrogène ou bien X' et Y représentent ensemble un groupe éthylène, la réaction étant effectuée par chauffage des réactifs dans un solvant convenable tel qu'un alcool, à une température voisine de la température d'ébullition dudit solvant et en présence d'un accepteur de l'acide chlorhydrique formé.

2. Procédé selon la revendication 1, caractérisé en ce que la réaction est effectuée en présence de cuivre utilisé comme catalyseur.

3. Procédé de préparation de dérivés de la méthyl-4 phényl-6 pyridazine de formule:

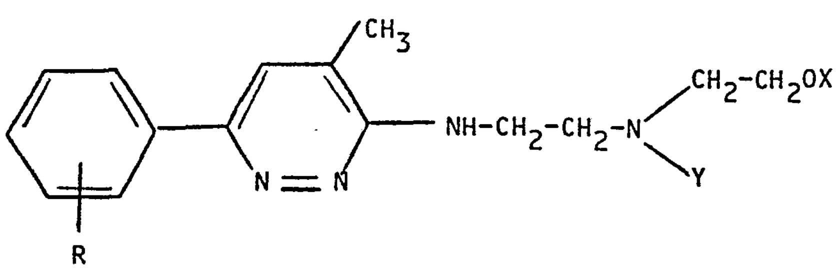

dans laquelle
R est H ou OH
X et Y représentent ensemble un groupe oxoéthylène, caractérisé en ce que l'on prépare un produit de formule (I), dans laquelle X et Y sont égaux à H en utilisant le procédé selon l'une des revendications 1 et 2 et que l'on fait réagir ledit produit sur un halogénure d'haloacétyle de formule

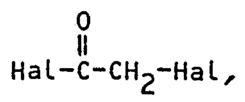

dans laquelle Hal est un halogène, ladite réaction étant effectuée dans un solvant inerte en présence d'un agent alcalin et à une température comprise entre -70 et 0°C et que l'on traite l'amine tertiaire obtenue en milieu méthanol avec un alcoolate alcalin.

4. Procédé de préparation de dérivés de la méthyl-4 phényl-6 pyridazine de formule:

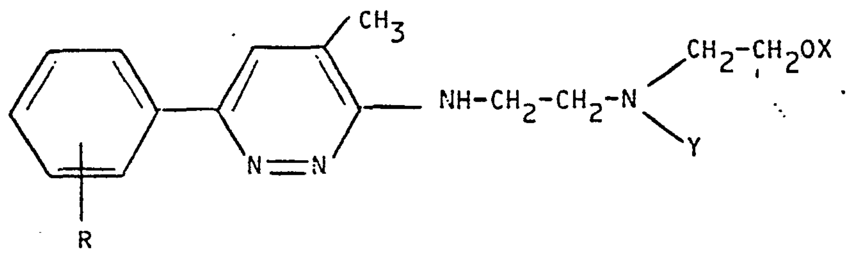

dans laquelle
R est H ou OH
X et Y représentent ensemble un groupe oxoéthylène, caractérisé en ce que l'on prépare un produit de formule (I), dans laquelle X et Y sont H selon l'une des revendications 1 et 2 et que l'on fait réagir ce produit avec le bromacétate d'éthyle, la réaction étant effectuée à reflux dans un solvant tel que le diméthylformamide et en présence d'une base organique telle que la triéthylamine et que l'on saponifie en milieu acide l'amine tertiaire obtenue.

5. Procédé de préparation de dérivés de la méthyl-4 phényl-6 pyridazine de formule:

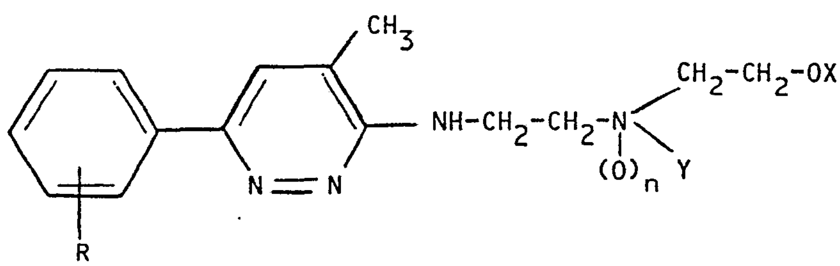

dans laquelle
R est H ou OH
n est 1
X et Y représentent ensemble un groupe éthylène, caractérisé en ce que l'on utilise comme produit de départ un produit de formule (I) préparé selon l'une des revendications 1 et 2. et que l'on traite ledit produit par un peracide tel que l'acide parachloroperbenzoïque, cette réaction étant effectuée dans un solvant inerte à une température inférieure à environ 20°C.

6. Procédé pour la préparation d'un dérivé de la méthyl-4 phényl-6 pyridazine de formule:

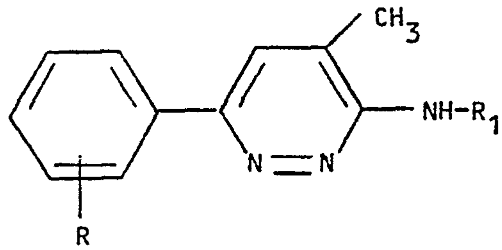

dans laquelle
R est H ou OH, et $R_1$ est H
caractérisé en ce que l'on fait réagir le produit de formule:

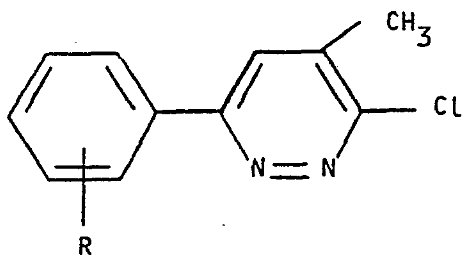

avec un large excès d'hydrazine de façon à obtenir le dérivé hydrazino-3 de la pyridazine et on hydrogène avec du nickel de Raney dans un solvant convenable le produit obtenu.

7. Utilisation des dérivés de la méthyl-4 phényl-6 pyridazine de formule

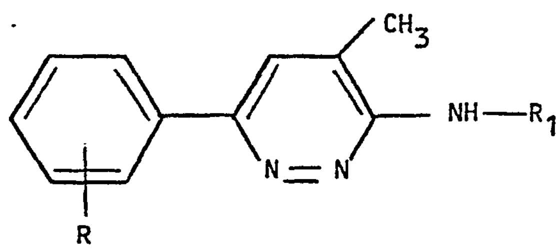

(I)

dans laquelle
R est H ou OH
$R_1$ représente H ou le groupe

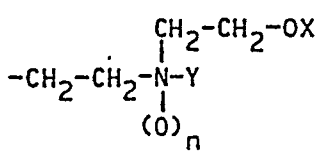

ou
n = 0 ou 1
X = H
Y = H ou alkyle inférieur (1 à 4 atomes de carbone)
ou bien X et Y pris ensemble forment un groupe éthylène ou oxoéthylène, avec les conditions suivantes:
1) lorsque n = 0 et X + Y est un groupe éthylène R est un groupe hydroxy;

2) lorsque n = 1 X + Y est un groupe éthylène, pour la préparation de médicaments utilisables notamment pour le traitement des états dépressifs.

## Patentansprüche

**für die Vertragsstaaten BE, CH, DE, FR, GB, IT, LI, LU, NL, SE**

1. 4-Methyl-6-phenyl-pyridazin-Derivate der Formel

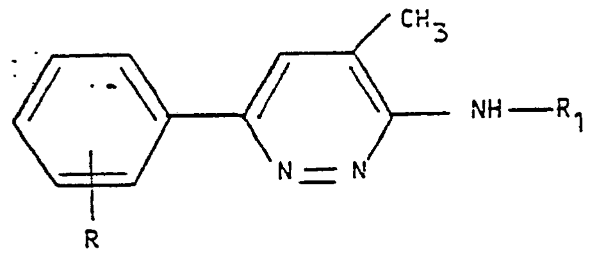

(I)

worin
R H oder OH ist,
$R_1$ H oder die Gruppe

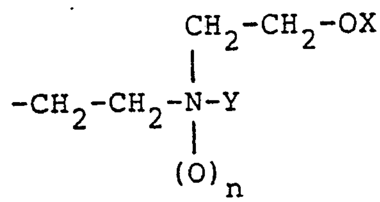

darstellt,
wobei
n = 0 oder 1
X = H
Y = H oder Niedrigalkyl (1 bis 4 Kohlenstoffatome) oder aber X und Y, zusammen genommen, eine Äthylen- oder Oxoäthylengruppe bilden, mit folgenden Maßgaben:
1) wenn n = 0 und X + Y eine Äthylengruppe ist, ist R eine Hydroxygruppe;
2) wenn n = 1, ist X + Y eine Äthylengruppe.

2. Derivate nach Anspruch 1, dadurch gekennzeichnet, daß sie die Additionssalze der Verbindungen der Formel (I) mit einer pharmazeutisch akzeptablen Säure sind.

3. Verfahren zur Herstellung von 4-Methyl-6-phenyl-pyridazin-Derivaten der Formel

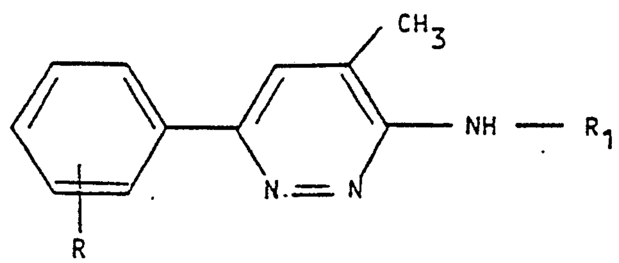

(I)

worin
R H oder OH ist
$R_1$ die Gruppe

$-CH_2CH_2N(CH_2CH_2OX)-Y$ darstellt,

darstellt,

wobei: X ist H, Y ist H oder Niedrigalkyl, oder aber X und Y stellen zusammen eine Äthylengruppe dar, wenn R für OH steht, dadurch gekennzeichnet, daß man in 6 durch eine gegebenenfalls substituierte Phenylgruppe substituiertes 3-Chlor-4-methyl-pyridazin mit einer Verbindung der Formel

$$NH_2-CH_2-CH_2-N(Y)-CH_2-CH_2-OX',$$

worin Y obige Bedeutung hat und X' für Wasserstoff steht oder aber X' und Y zusammen eine Äthylengruppe darstellen, reagieren läßt, welche Reaktion durch Erhitzen der Reagenzien in einem geeigneten Lösungsmittel, wie einem Alkohol, auf eine Temperatur nahe der Kochtemperaturen des Lösungsmittels und in Gegenwart eines Akzeptors für die entstandene Salzsäure durchgeführt wird.

4. Verfahren nach Anspruch 3, dadurch gekennzeichnet, daß die Reaktion in Gegenwart von Kupfer als Katalysator durchgeführt wird.

5. Verfahren zur Herstellung von 4-Methyl-6-phenyl-pyridazin-Derivaten der Formel

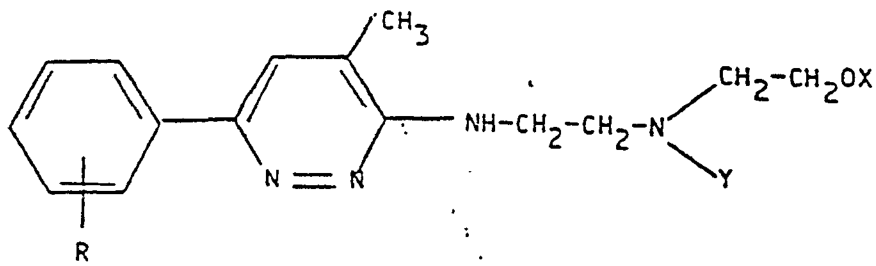

worin

R für H oder OH steht,

X und Y zusammen eine Oxoäthylengruppe darstellen, dadurch gekennzeichnet, daß man unter Anwendung des Verfahrens nach einem der Ansprüche 3 und 4 ein Produkt der Formel (I), worin X und Y gleich H sind, herstellt und das Produkt mit einem Halogenessigsäurehalogenid der Formel

$$Hal-C(=O)-CH_2-Hal,$$

worin Hal für ein Halogen steht, reagieren läßt, welche Reaktion in einem inerten Lösungsmittel in Gegenwart eines alkalischen Mittels und bei einer Temperatur zwischen -10 und 0°C durchgeführt wird, und daß man das erhaltene tertiäre Amin in Methanolmilieu mit einem Alkalialkoholat behandelt.

6. Verfahren zur Herstellung von 4-Methyl-6-phenyl-pyridazin-Derivaten der Formel

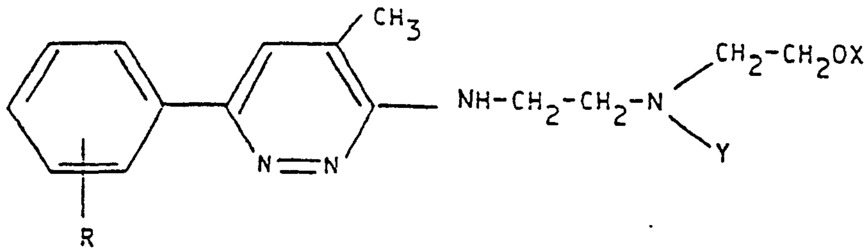

worin R für H oder OH steht,
X und Y zusammen eine Oxoäthylengruppe darstellen, dadurch gekennzeichnet, daß man ein Produkt der Formel (I), worin X und Y für H stehen, nach einem der Ansprüche 3 und 4 herstellt, und daß man dieses Produkt mit Äthylbromacetat reagieren läßt, welche Reaktion unter Rückfluß in einem Lösungsmittel, wie Dimethylformamid, und in Gegenwart einer organischen Base, wie Tritäthylamin, durchführt, und daß man das erhaltene tertiäre Amin in saurem Milieu verseift.

7. Verfahren zur Herstellung von 4-Methyl-6-phenyl-pyridazin-Derivaten der Formel

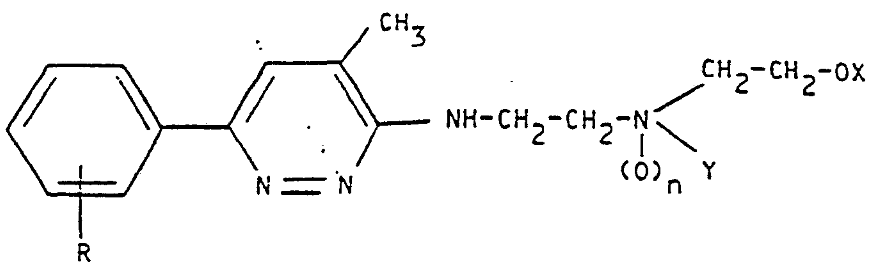

worin
R für H oder OH steht,
n 1 ist,
X und Y zusammen eine Äthylengruppe darstellen, dadurch gekennzeichnet, daß man als Ausgangsprodukt ein nach einem der Ansprüche 3 und 4 hergestelltes Produkt der Formel (I) verwendet, und daß man das Produkt mit einer Persäure, wie Parachlorperbenzoesäure behandelt, welche Reaktion in einem inerten Lösungsmittel bei einer Temperatur von weniger als etwa 20° C durchgeführt wird.

8. Verfahren zur Herstellung eines 4-Methyl-6-phenyl-pyridazin-Derivats der Formel

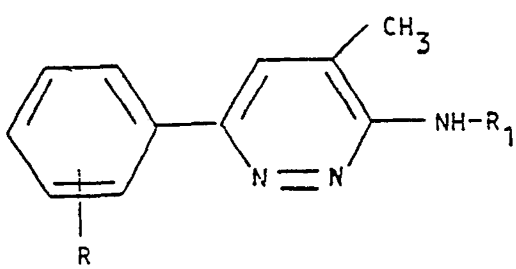

worin
R für H oder OH steht und $R_1$ H bedeutet, dadurch gekennzeichnet, daß man das Produkt der Formel

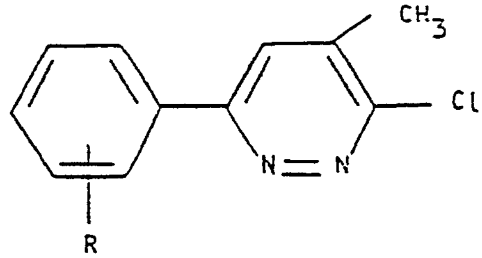

mit einem großen Überschuß an Hydrazin derart reagieren läßt, daß man das 3-Hydrazino-pyridazin-Derivat erhält, und man das erhaltene Produkt in einem geeigneten Lösungsmittel mit Raney-Nickel hydriert.

9. Medikamente, die insbesondere zur Behandlung von Depressionszuständen geeignet sind, dadurch gekennzeichnet, daß sie mindestens ein Produkt nach einem der Ansprüche 1 und 2 enthalten.

10. Medikamente nach Anspruch 9, dadurch gekennzeichnet, daß sie 0,01 g bis 0,500 g mindestens eines Produkts nach den Ansprüchen 1 und 2 enthalten.

**Patentansprüche**

**für den Vertragsstaat AT**

1. Verfahren zur Herstellung von 4-Methyl-6-phenyl-pyridazin-Derivaten der Formel

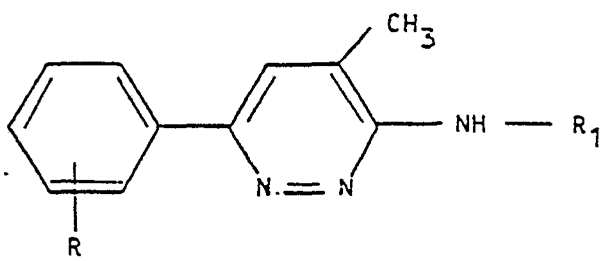

(I)

worin
R H oder OH ist
$R_1$ die Gruppe

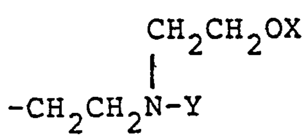

darstellt, in welcher X H ist, Y H oder Niedrigalkyl darstellt oder aber X und Y zusammen eine Äthylengruppe bilden, wenn R für OH steht, dadurch gekennzeichnet, daß man in 6 durch eine gegebenenfalls substituierte Phenylgruppe substituiertes 3-Chlor-4-methyl-pyridazin mit einer Verbindung der Formel

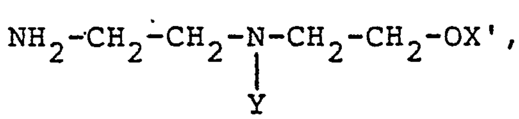

worin Y obige Bedeutung hat und X' für Wasserstoff steht oder aber X' und Y zusammen eine Äthylengruppe darstellen, reagieren läßt, welche Reaktion durch Erhitzen der Reagenzien in einem geeigneten Lösungsmittel,

wie einem Alkohol, auf eine Temperatur nahe der Kochtemperatur des Lösungsmittels und in Gegenwart eines Akzeptors für die entstandene Salzsäure durchgeführt wird.

2. Verfahren nach Anspruch 1, dadurch gekennzeichnet, daß die Reaktion in Gegenwart von Kupfer als Katalysator durchgeführt wird.

3. Verfahren zur Herstellung von 4-Methyl-6-phenyl-pyridazin-Derivaten der Formel

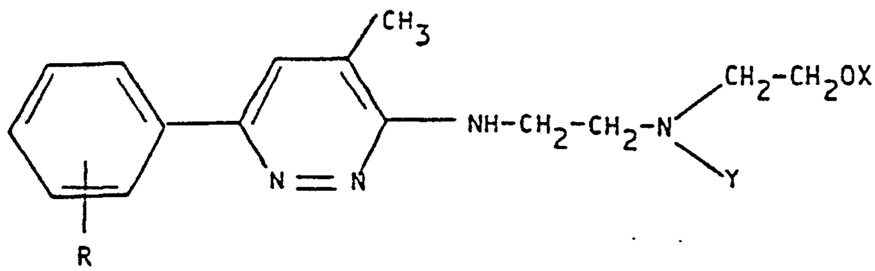

worin
R für H oder OH steht,
X und Y zusammen eine Oxoäthylengruppe darstellen, dadurch gekennzeichnet, daß man unter Anwendung des Verfahrens nach einem der Ansprüche 1 und 2 ein Produkt der Formel (I), worin X und Y gleich H sind, herstellt und das Produkt mit einem Halogenessigsäurehalogenid der Formel

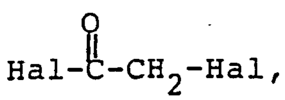

worin Hal für ein Halogen steht, reagieren läßt, welche Reaktion in einem inerten Lösungsmittel in Gegenwart eines alkalischen Mittels und bei einer Temperatur zwischen -10 und 0°C durchgeführt wird, und daß man das erhaltene tertiäre Amin in Methanolmilieu mit einem Alkalialkoholat behandelt.

4. Verfahren zur Herstellung von 4-Methyl-6-phenyl-pyridazin-Derivaten der Formel

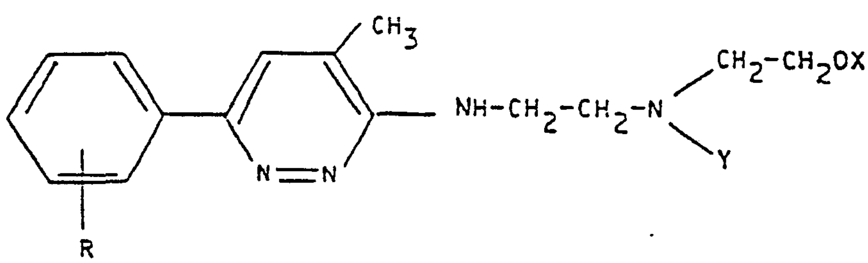

worin R für H oder OH steht,
X und Y zusammen eine Oxoäthylengruppe darstellen, dadurch gekennzeichnet, daß man ein Produkt der Formel (I), worin X und Y für H stehen, nach einem der Ansprüche 1 und 2 herstellt, und daß man dieses Produkt mit Äthylbromacetat reagieren läßt, welche Reaktion unter Rückfluß in einem Lösungsmittel, wie Dimethylformamid, und in Gegenwart einer organischen Base, wie Tritäthylamin, durchführt, und daß man das erhaltene tertiäre Amin in saurem Milieu verseift.

5. Verfahren zur Herstellung von 4-Methyl-6-phenyl-pyridazin-Derivaten der Formel

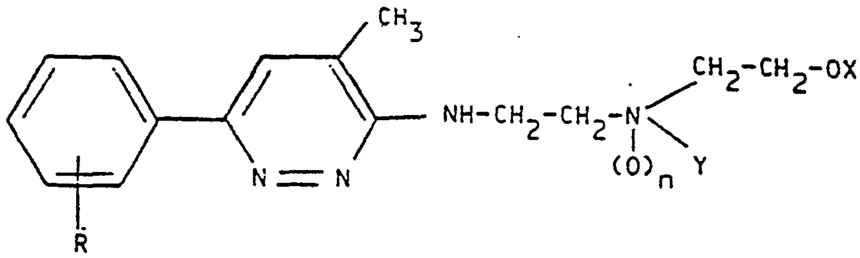

worin
R für H oder OH steht,
n 1 ist,
X und Y zusammen eine Äthylengruppe darstellen, dadurch gekennzeichnet, daß man als Ausgangsprodukt ein nach einem der Ansprüche 1 und 2 hergestelltes Produkt der Formel (I) verwendet, und daß man das Produkt mit einer Persäure, wie Parachlorperbenzoesäure behandelt, welche Reaktion in einem inerten Lösungsmittel bei einer Temperatur von weniger als etwa 20° C durchgeführt wird.

6. Verfahren zur Herstellung eines 4-Methyl-6-phenyl-pyridazin-Derivats der Formel

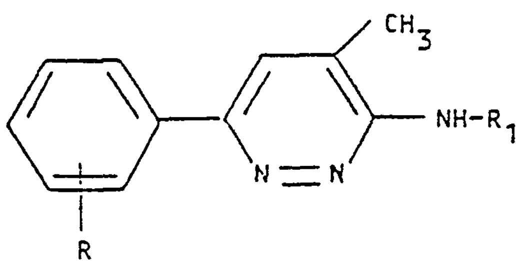

worin
R für H oder OH steht und $R_1$ H bedeutet, dadurch gekennzeichnet, daß man das Produkt der Formel

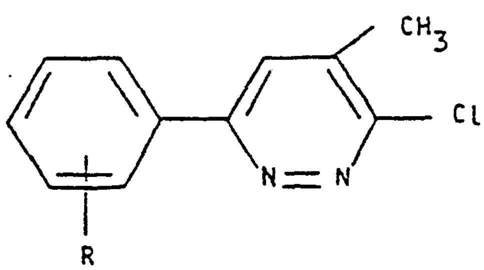

mit einem großen Uberschuß an Hydrazin derart reagieren läßt, daß man das 3-Hydrazino-pyridazin-Derivat erhält, und man das erhaltene Produkt in einem geeigneten Lösungsmittel mit Raney-Nickel hydriert.

7. Verwendung der 4-Methyl-6-phenyl-pyridazin-Derivate der Formel

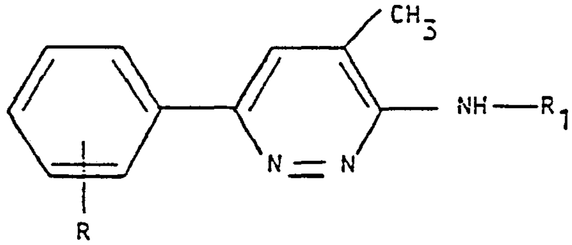

(I)

worin
R H oder OH ist,
$R_1$ H oder die Gruppe

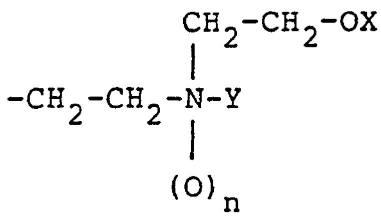

darstellt,
wobei
n = 0 oder 1
X = H
Y = H oder Niedrigalkyl (1 bis 4 Kohlenstoffatome) oder aber X und Y, zusammen genommen, eine Äthylen- oder Oxoäthylengruppe bilden, mit folgenden Maßgaben:
1) wenn n = 0 und X + Y eine Äthylengruppe ist, ist R eine Hydroxygruppe,;
2) wenn n = 1, ist X + Y eine Äthylengruppe, zur Herstellung von Medikamenten, die insbesondere zur Behandlung von Depressionszuständen geeignet sind.

**Claims**

**for the contracting States: BE, CH, DE, FR, GB, IT, LI, LU, NL, SE**

1. 4-methyl-6-phenyl-pyridazine derivatives of formula :

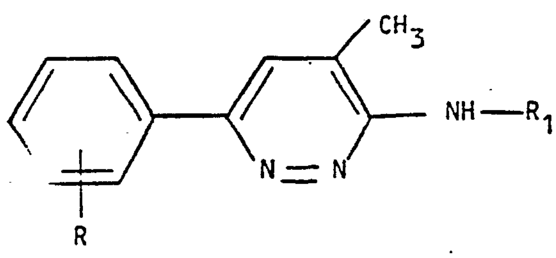

(I)

in which
R is H or OH
$R_1$ represents H or the

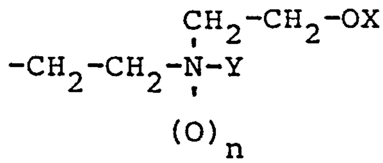

group
wherein
n = 0 or 1
X = H
Y = H or a lower alkyl (1 to 4 atoms of carbon) or X and Y taken together form an ethylene or oxoethylene group, with the following conditions:
1) when n = 0 and X + Y is an ethylene group, R is a hydroxy group;
2) when n = 1 X + Y is an ethylene group.

2. Derivatives according to claim 1, characterized in that they are the addition salts of the compounds of formula (I) with a pharmaceutically acceptable acid.

3. Process for preparing 4-methyl-6-phenyl-pyridazine of formula:

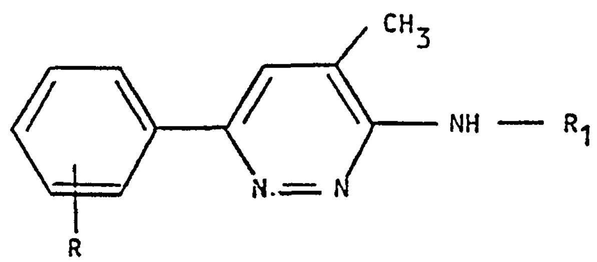

(I)

in which
R is H or OH
$R_1$ represents the

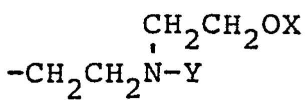

group,
in which: X is H, Y is H or a lower alkyl or
X and Y represent together an ethylene group when R is OH, characterized in that the 3-chloro 4-methyl pyridazine substituted in 6 by an optionally substituted phenyl group is reacted with a compound of formula:

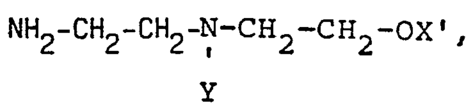

in which Y is such as defined above, and X' represent hydrogen or X' and Y represent together an ethylene group, the reaction being carried out by heating reagents in asuitable solvent such as an alcohol, at a temperature close to the boiling temperature of said solvent and in the presence of an acceptor of the formed hydrochloric acid.

4. Process according to claim 3, characterized in that the reaction is carried out in the presence of copper used as catalyst.

5. Process for preparing derivatives of 4-methyl 6-pyridazine of formula:

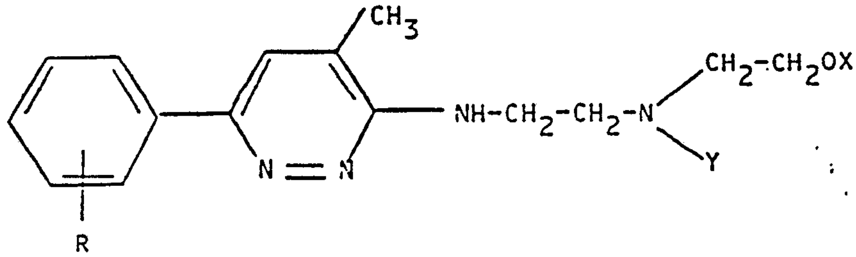

in which
R is H or OH
X and Y represent together an oxoethylene group, characterized in that a product of formula (I) in which X and Y are equal to H, is prepared by using the process according to any one of claims 3 and 4, and in that said product is reacted on a haloacetyl halide of formula

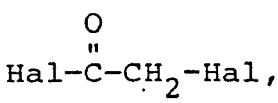

in which Hal is a halogen, said reaction being carried out in an inert solvent in the presence of an alkaline agent and at a temperature of between -10' and 0°C and in that the tertiary amine obtained in methanol medium is treated with an alkaline alcoholate.

6. Process for preparing derivatives of 4-methyl-6-phenyl-pyridazine of formula:

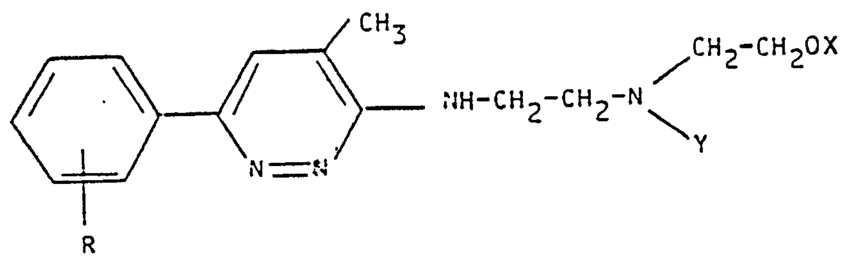

in which
R is H or OH
X and Y represent together an oxoethylene group, characterized in that a product of formula (I) in which X and Y are H is prepared according to any one of claims 3 and 4 and in that this product is reacted with ethyl bromacetate, the reaction being effected at reflux in a solvent such as dimethylformamide and in the presence of an organic base such as triethylamine and the tertiary amine thus obtained is saponified in acid medium.

7. Process for preparing derivatives of 4-methyl-6-phenyl-pyridazine of formula:

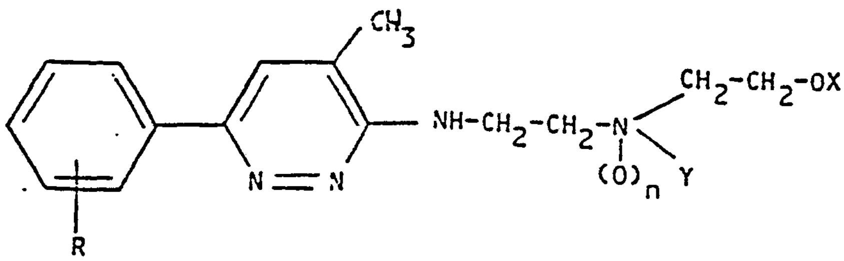

in which
R is H or OH
n is 1
X and Y represent together an ethylene group, characterized in that a product of formula (I) prepared according to any one of claims 3 and 4 is used, and in that said product is treated with a peracid such as parachloroperbenzoic acid, this reaction being effected in an inert solvent at a temperature lower than 20° C.

8. Process for preparing a derivative of 4-methyl-6-phenyl-pyridazine of formula:

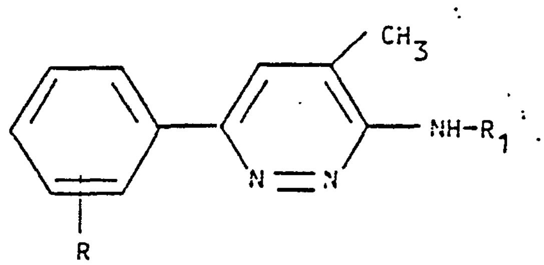

in which
R is H or OH, and $R_1$ is H
characterized in that the product of formula:

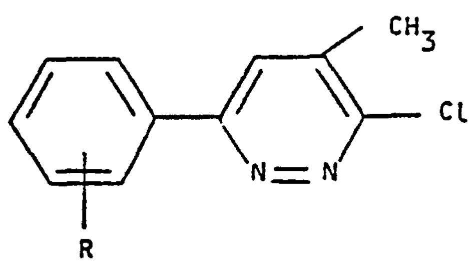

is reacted with a large excess of hydrazine so as to obtain the 3-hydrazino derivative of pyridazine and the product thus obtained is hydrogenated with Raney nickel in an appropriate solvent.

9. Drugs usable in particular in the treatment of depressive states, characterized in that they contain at least one product according to any one of claims 1 and 2.

10. Drugs according to claim 9, characterized in that they contain from 0.01 g to 0.500 g of at least one product according to claims 1 and 2.

**Claims**

**for the contracting State: AT**

1. Process for preparing 4-methyl-6-phenyl-pyridazine derivatives of formula:

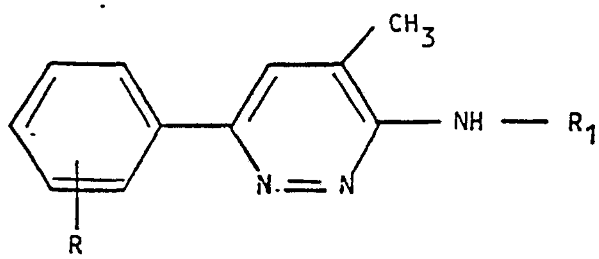

(I)

in which
R is H or OH
$R_1$ represents the

$CH_2CH_2OX$
$-CH_2CH_2N-Y$

group in which:
X is H, Y is H or a lower alkyl or X and Y represent together an ethylene group when R is OH, characterized in that the 3-chloro 4-methyl pyridazine substituted in 6 by an optionally substituted phenyl group with a compound of formula

$NH_2-CH_2-CH_2-N-CH_2-CH_2-OX'$,
$Y$

in which Y is such as defined above, and X' represents hydrogen or X' and Y represent together an ethylene group, the reaction being carried out by heating reagents in a suitable solvent such as an alcohol, at a temperature close to the boiling temperature of said solvent and in the presence of an acceptor of the formed hydrochloric acid.

2. Process according to claim 1, characterized in that the reaction is carried out in the presence of copper used as catalyst.

3. Process for preparing 4-methyl 6-phenyl-pyridazine formula:

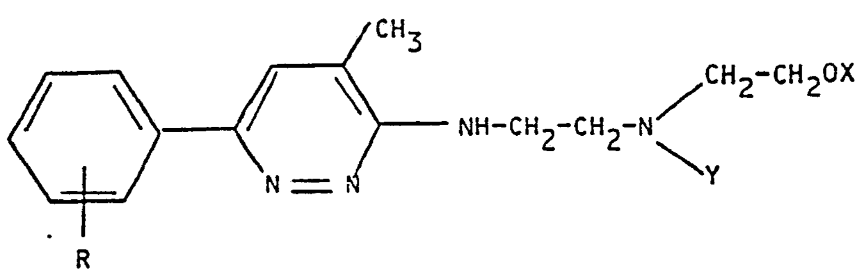

in which R is H or OH
X and Y represent together an oxoethylene group, characterized in that a product of formula (I) in which X and Y are equal to H, is prepared by using the process according to any one of claims 1 and 2, and in that said product is reacted on a haloacetyl halogen of formula

$$Hal-\overset{O}{\overset{\|}{C}}-CH_2-Hal,$$

in which Hal is a halogen, said reaction being carried out in an inert solvent in the presence of an alkaline agent and at a temperature of between -10° and 0°C and in that the tertiary amine obtained in methanol medium is treated with an alkaline alcoholate.

4. Process for preparing derivatives of 4-methyl-6-phenyl-pyridazine of formula:

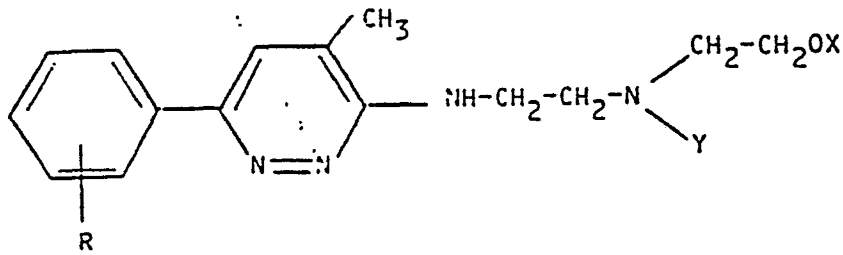

in which
R is H or OH
X and Y represent together an oxoethylene group, characterized in that a product of formula (I) in which X and Y are H is prepared according to any one of claims 1 and 2 and in that this product is reacted with ethyl bromacetate, the reaction being effected at reflux in a solvent such as dimethylformamide and in the presence of an organic base such as triethylamine and the tertiary amine thus obtained is saponified in acid medium.

5. Process for preparing derivatives of 4-methyl-6-phenyl-pyridazine of formula:

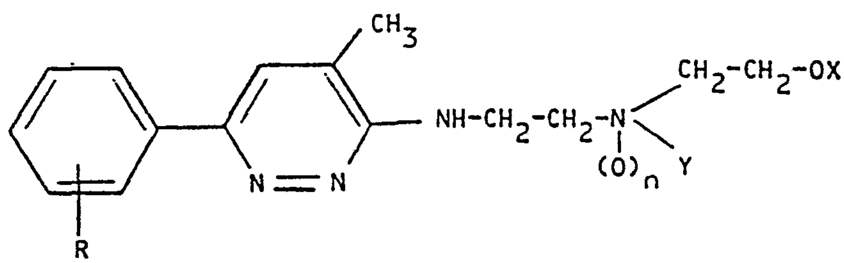

in which
R is H or OH
n is 1
X and Y represent together an ethylene group, characterized in that a product of formula (I) prepared according to any one of claims 1 and 2 is used, and in that said product is treated with a peracid such as parachloroperbenzoic acid, this reaction being effected in an inert solvent at a temperature lower than 20°C.

6. Process for preparing a derivative of 4-methyl-6-phenyl-pyridazine of formula:

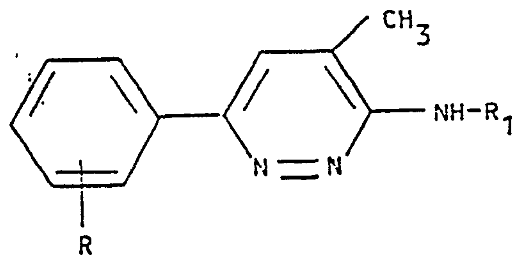

in which
R is H or OH, and $R_1$ is H
characterized in that the product of formula:

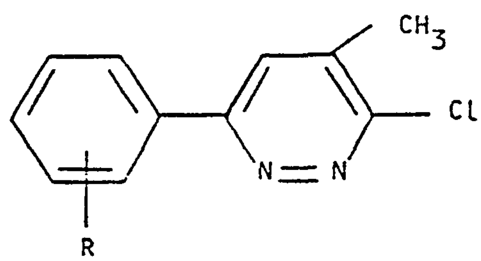

is reacted with a large excess of hydrazine so as to obtain the 3-hydrazine derivatives of pyridazine acid the product thus obtained is hydrogenated with Raney nickel in an appropriate solvent.

7. Use of 4-methyl-6-phenyl-pyridazine derivatives of formula

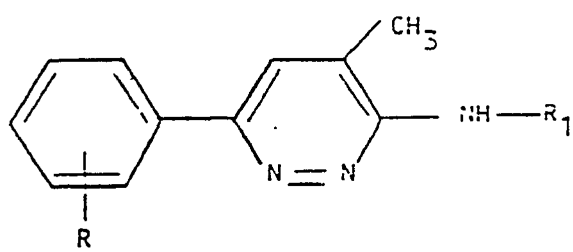

(I)

for the preparation of drugs usable in particular in the treatment of depressive states,
in which
R is H or OH
$R_1$ represents H or the

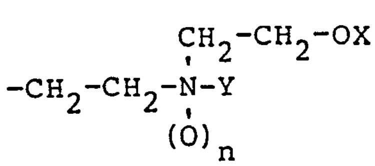

group
wherein n = 0 or 1
X = H

Y = H or a lower alkyl (1 to 4 atoms of carbon)
or X and Y taken together form an ethylene or oxoethylene group, with the following conditions:
1) when n = 0 and X + Y is an ethylene group, R is is a hydroxy group;
2) when n = 1,X + Y is an ethylene group.